(19) Europäisches Patentamt
European Patent Office
Office européen des brevets

(11) **EP 4 179 298 B1**

(12) **EUROPEAN PATENT SPECIFICATION**

(45) Date of publication and mention
of the grant of the patent:
**17.07.2024   Bulletin 2024/29**

(21) Application number: **21751617.8**

(22) Date of filing: **08.07.2021**

(51) International Patent Classification (IPC):
**G01N 21/65** [(2006.01)]

(52) Cooperative Patent Classification (CPC):
**G01N 21/65**

(86) International application number:
**PCT/IB2021/056114**

(87) International publication number:
**WO 2022/009127 (13.01.2022 Gazette 2022/02)**

(54) **INFLAMMATORY BOWEL DISEASE DIAGNOSIS METHOD**

DIAGNOSEVERFAHREN FÜR ENTZÜNDLICHE DARMERKRANKUNGEN

MÉTHODE DE DIAGNOSTIC DE MALADIES INFLAMMATOIRES DE L'INTESTIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **09.07.2020   IT 202000016714**

(43) Date of publication of application:
**17.05.2023   Bulletin 2023/20**

(73) Proprietor: **IRCCS Centro Neurolesi "Bonino-Pulejo"
98124 Messina (IT)**

(72) Inventors:
• **ACRI, Giuseppe
  87045 Dipignano (IT)**
• **COSTA, Stefano
  98125 Messina (IT)**
• **TESTAGROSSA, Barbara
  98168 Messina (IT)**
• **VENUTI, Valentina
  98168 Messina (IT)**
• **CRUPI, Vincenza
  98165 Messina (IT)**
• **MAJOLINO, Domenico
  96168 Messina (IT)**
• **CIURLEO, Rosella
  89050 Feroleto della Chiesa (IT)**
• **DENARO, Fabrizio
  98121 Messina (IT)**
• **BRAMANTI, Alessia
  98121 Messina (IT)**

(74) Representative: **Celona, Antonio et al
Notarbartolo & Gervasi S.p.A.
Viale Achille Papa, 30
20149 Milano (IT)**

(56) References cited:
**WO-A1-2005/017501     WO-A2-2013/132338**

• **PENCE ISAAC J. ET AL: "Clinical characterization of in vivo inflammatory bowel disease with Raman spectroscopy", BIOMEDICAL OPTICS EXPRESS, vol. 8, no. 2, 4 January 2017 (2017-01-04), United States, pages 524, XP055789259, ISSN: 2156-7085, DOI: 10.1364/BOE.8.000524**
• **MORASSO CARLO: "Raman Analysis Reveals Biochemical Differences in Plasma of Crohn's Disease Patients", 28 April 2020 (2020-04-28), XP055789395, Retrieved from the Internet <URL:https://academic.oup.com/ecco-jcc/article-abstract/14/11/1572/5826348>**
• **ACRI GIUSEPPE ET AL: "Raman Spectroscopy as Noninvasive Method of Diagnosis of Pediatric Onset Inflammatory Bowel Disease", APPLIED SCIENCES, vol. 10, no. 19, 5 October 2020 (2020-10-05), pages 6974, XP055789186, DOI: 10.3390/app10196974**

EP 4 179 298 B1

## Description

Field of the invention

The present invention relates to a method for diagnosing and possibly differentiating inflammatory bowel diseases such as Ulcerative Colitis (UC) and Crohn's Disease (CD), in particular with pediatric onset.

Background art

An early and accurate diagnosis of chronic diseases is crucial in view of an adequate intervention. It can also help facilitate the monitoring of disease progression and therapeutic response. In many chronic diseases, especially in relation to pediatric patients, the diagnostic process can be time consuming and invasive, thus the search for non-invasive and accurate diagnostic tools is ongoing.

For example, diagnosing inflammatory bowel disease (IBD), in particular in childhood, can be difficult. Such a diagnosis is based on the detection of chronic inflammation in the gastrointestinal (GI) tract and the exclusion of other causes of inflammation. In particular, the differentiation of Crohn's disease (CD) from ulcerative colitis (UC), as well as of both of the aforementioned diseases from infectious diseases, allergic diseases or primary immunodeficiency disorders (PID) with similar manifestations, is based on a combination of clinical suspicion, endoscopic and histological evaluation of the mucosa and other additional tests in the event of uncertainty. An international group of European experts in pediatric inflammatory bowel disease (PIBD), mainly from the "Porto" IBD working group of the European Society of Pediatric Gastroenterology, Hepatology, and Nutrition (ESPGHAN), has said that an "accurate diagnosis of inflammatory bowel disease should be based on a combination of history, physical and laboratory examination, esophagogastroduodenoscopy (EGD) and ileocolonoscopy with histology, as well as imaging of the small intestine. This is essential for excluding enteric infections". While adhering to the revised diagnosis criteria of the "Porto" IBD working group, in some cases distinguishing between Crohn's disease (CD) and ulcerative colitis (UC), and distinguishing both of these diseases from other diseases is somewhat problematic, especially in the case of patients with early disease onset.

The development of a non-invasive investigation methodology which is useful, on the one hand, in reducing the burden of diagnosis and, on the other hand, in differentiating between CD and UC, is a goal for current and future research. However, structural tissue analysis has not proved to be effective in differentiating inflammation subtypes. Alternative methodologies have also been attempted, but without obtaining satisfactory results.

In this context, it is evident from the large existing literature that many inflammatory conditions have also been studied by means of Raman spectroscopy.

From a general point of view, Raman spectroscopy is an inelastic light scattering phenomenon according to which the illumination of a molecule by monochromatic laser light will give rise to an exchange of vibrational energy, which will result in a difference in the vibrational frequency between incident beam and scattered beam. Consequently, this experimental method provides a vibrational spectrum which contains information relating to the chemical bonds and symmetry of a specific molecule. Such a technique forms an essential, non-invasive analysis methodology on a molecular scale in physics, chemistry, biology and materials science, recently finding increasingly wide application also in the field of clinical diagnostics.

For example, a Raman probe coupled with an endoscope was used *in vivo* to study the composition of the colon mucosa in patients with ulcerative colitis, for whom a decrease in phosphatidylcholine (720 cm$^{-1}$) and total lipids (1303 cm$^{-1}$) was observed. In another example, Raman spectroscopy based on the use of an optical fiber probe in colonoscopy was applied *in vivo* for the diagnosis, in real time and in a non-destructive manner, of inflammatory bowel diseases such as ulcerative colitis and Crohn's disease but, since the probe is mounted on an optical fiber, the methodology has always proved to be completely invasive.

Therefore, disadvantageously, in most cases the current background art does not allow to make a certain diagnosis of inflammatory bowel diseases, such as UC and CD, in a non-invasive manner, in particular without performing at least a duodenal biopsy and/or other *in vivo* analysis.

The article: PENCE ISAAC J. ET AL: "Clinical characterization of in vivo inflammatory bowel disease with Raman spectroscopy", BIOMEDICAL OPTICS EXPRESS, vol. 8, no. 2, 4 January 2017, pages 524-535, discloses Raman spectroscopy of colon tissue with an endoscope to detect inflammatory Bowel disease, distinguishing between colitis ulcerosa and Crohn's disease. A spectral band corresponding to an amide-I vibration mode (1658 cm$^{-1}$) is considered.

Summary of the invention

An object of the present invention is to provide a highly efficient and non-invasive automated diagnosis method of some inflammatory bowel diseases, comprising

Ulcerative Colitis (UC) and Crohn's Disease (CD), which can always avoid duodenal biopsy.

A further object of the present invention is to provide an alternative diagnosis method of said inflammatory bowel diseases, based on the spectroscopic analysis by Raman technique, which operates directly on a protein extract of feces. Another object of the present invention is to provide a non-invasive diagnostic method which reduces the burden of diagnosis while at the same time being useful for the automated distinction between Ulcerative Colitis (UC) and Crohn's Disease (CD).

The present invention achieves the above objects by providing a diagnosis method of inflammatory bowel diseases, comprising Ulcerative Colitis (UC) and Crohn's Disease (CD), according to claim 1.

With the aim of identifying a procedural path which takes into consideration only a physical investigation technique, such as Raman spectroscopy, a methodology has been developed, based on the analysis by Raman spectrometer of a protein extract of patients' feces, to diagnose and possibly differentiate inflammatory bowel diseases (IBD), comprising ulcerative colitis (UC) and Crohn's disease (CD), in particular with pediatric onset, in a completely non-invasive manner without performing any duodenal biopsy.

In particular, the Raman technique has been applied advantageously to the protein extract of fecal samples in order to obtain information on molecular vibrations which can potentially provide spectral markers of cellular modifications which occur following specific pathological conditions. Attention was focused on investigating the amide-I vibration region, quantitatively evaluating the spectral changes in the protein secondary structures by applying deconvolution and curve-fitting.

It was surprisingly found that inflammation causes a significant increase in the non-reducible (trivalent)/reducible (bivalent) cross-linking ratio R of the protein network. This parameter has proved to be an excellent marker for distinguishing individuals with IBD from non-IBD individuals and also making automatic the distinction, among patients with IBD, between subjects suffering from UC and subjects suffering from CD. The proposed methodology was fully validated by statistical analysis using the ROC (Receiver Operating Characteristic) curve.

The new methodology, object of the present invention, has several advantages:

- the technique is not invasive;
- extremely low cost, with respect to the currently used diagnostic techniques, which require the use of specific diagnostic kits;
- simplicity of use, experimental repeatability, and speed of execution;
- the technique is not destructive; in fact, the sample in question can be stored and analyzed several times over time, since the only limit of the analysis is given by the degradation of the sample itself.

Further features and advantages of the invention will become apparent in light of the detailed description of some exemplary but not exclusive embodiments.

The dependent claims describe particular embodiments of the invention.

Brief description of the drawings

In the description of the invention, reference is made to the accompanying drawings, which are provided by way of non-limiting example, in which:

Figure 1a shows an example of an average Raman spectrum in the region 1300-1800 cm$^{-1}$ of the fecal protein extract of non-IBD subjects (black line), subjects with CD (red line) and subjects with UC (green line);

Figure 1b shows an example of a profile obtained by calculating the second derivative of the band comprised in a wavenumber range of 1550-1750 cm$^{-1}$;

Figure 2a shows best-fit results for the region 1550-1750 cm$^{-1}$ of a sample related to a non-IBD patient and the related sub-bands;

Figure 2b shows best-fit results for the region 1550-1750 cm$^{-1}$ of a sample related to a patient with CD and the related sub-bands;

Figure 2c shows best-fit results for the region 1550-1750 cm$^{-1}$ of a sample related to a patient with UC and the related sub-bands;

Figure 3 shows the calculated values of the ratio R for non-IBD subjects (black circles), subjects with CD (red squares) and subjects with UC (green triangles);

Figure 4 shows an ROC curve for non-IBD and IBD subjects from a sample of patients;

Figure 5 shows the best cutoff-point to maximize sensitivity and specificity for said non-IBD and IBD subjects;

Figure 6 shows the distribution graph for said non-IBD and IBD subjects;

Figure 7 shows an ROC curve for the group of IBD subjects of said patient sample;

Figure 8 shows the best cutoff-point to maximize sensitivity and specificity for said IBD subjects;

Figure 9 shows the distribution graph for UC and CD subjects belonging to said group of IBD subjects.

Detailed description of embodiments of the invention

The method of the invention for the diagnosis of inflammatory bowel diseases, comprising Ulcerative Colitis (UC) and Crohn's Disease (CD), comprises the following steps:

a) providing, as input data, a Raman spectrum of a protein extract of a fecal sample;

b) isolating, from said Raman spectrum, the band in a wavenumber range of about 1550-1750 cm$^{-1}$, which defines the amide-I vibration modes;

c) calculating the second derivative profile of said Raman spectrum band, and identifying the minima of said profile;

d) performing a deconvolution of said band, setting a number of sub-bands equal to the number of minima of the profile of said second derivative, obtaining a number of sub-bands equal to at least said number of minima, each sub-band having a respective cent-

er-frequency $\omega_i$ and a respective area $A_i$, with i = 1, 2, ... n;

e) associating the sub-bands obtained in step d), based on the center-frequency $\omega_i$ thereof, with the corresponding vibration modes of the protein secondary structures comprising at least the vibration modes of the disordered protein secondary structures of amide-I and the vibration modes of the higher frequency anti-parallel β-sheet protein secondary structures;

f) calculating the ratio R of the area of the sub-band, associated with the vibration modes of the disordered protein secondary structures of amide-I, with the area of the sub-band associated with the vibration modes of the higher frequency anti-parallel β-sheet protein secondary structures;

g) verifying that the ratio R is greater than or equal to a first threshold value to confirm that the protein extract of the fecal sample belongs to a patient suffering from an inflammatory bowel disease between Ulcerative Colitis (UC) and Crohn's Disease (CD).

For example, the vibration modes of the disordered protein secondary structures of amide-I have the respective center-frequency at a wavenumber in the range 1660-1689 cm$^{-1}$, for example about 1662 cm$^{-1}$, compatibly with the resolution of the Raman spectrometer; while the vibration modes of the higher frequency anti-parallel β-sheet protein secondary structures have the respective center-frequency at a wavenumber in the range 1690-1700 cm$^{-1}$, for example about 1694 cm$^{-1}$, compatibly with the resolution of the Raman spectrometer.

Other significant vibration modes which can fall within the band comprised in the wavenumber range of about 1550-1750 cm$^{-1}$ are:

- the vibration modes of the parallel β-sheet protein secondary structures, which have the respective center-frequency at a wavenumber in the range 1610-1619 cm$^{-1}$, compatibly with the resolution of the Raman spectrometer,
- the vibration modes of the lower frequency anti-parallel β-sheet protein secondary structures, which have the respective center-frequency at a wavenumber in the range 1620-1639 cm$^{-1}$, always compatibly with the resolution of the Raman spectrometer,
- and the vibration modes of the α-helix protein secondary structures, which have the respective center-frequency at a wavenumber in the range 1640-1654 cm$^{-1}$, always compatibly with the resolution of the Raman spectrometer.

Preferably, it is possible to automatically distinguish whether the ill patient is suffering from Ulcerative Colitis (UC) or Crohn's Disease (CD). In fact, if the above ratio R is greater than or equal to a second threshold value it can be confirmed that the protein extract of the fecal sample belongs to a patient suffering from Ulcerative Colitis (UC). Otherwise, if the ratio R is less than the second threshold value, it can be confirmed that the protein extract of the fecal sample belongs to a patient with Crohn's disease (CD).

The first threshold value can be defined by performing the following steps:

- providing, as input data, a Raman spectrum of a fecal sample protein extract for each patient of a first known group of patients with Ulcerative Colitis (UC) or Crohn's Disease (CD) and for each patient of a second known group of healthy patients;
- for each patient, of both the first group and second group, isolating from the respective Raman spectrum the band in the wavenumber range of about 1550-1750 cm$^{-1}$;
- performing the steps c), d), e) and f) described above for each Raman spectrum;
- performing an analysis of a first ROC curve considering the ratios R of the first group and of the second group as database and calculating the first threshold value by Youden's index.

As is known, most diagnostic tests produce a quantitative result. To discriminate between healthy and ill people it is necessary to have a threshold or cut-off value. In an ideal situation, healthy and ill people return different test values and the cut-off value is immediately determined. In real situations, there is always some overlap in the distribution of healthy and ill people. Sensitivity and specificity are inversely related in relation to the selection of the cut-off. The adoption of a threshold which offers high sensitivity leads to a loss of specificity and vice versa.

In the proposed method, although the distribution of healthy and ill people is distinct enough, to obtain the threshold value which minimizes the probability of finding false positives and false negatives, it is preferable to construct a ROC (Receiver Operating Characteristic) curve in a known manner, with sensitivity on the ordinates and (1-specificity) on the abscissas, considering the ratios R as the database, therefore obtaining the first optimal threshold value. Accuracy, sensitivity and specificity of the proposed diagnostic test were determined using said ROC curve and Youden's index, providing excellent results.

The second threshold value can instead be defined by performing the following steps:

- providing, as input data, a Raman spectrum of a fecal sample protein extract for each patient of a first known subgroup of said first group consisting of patients with Ulcerative Colitis (UC) and for each patient of a second known subgroup of said first group consisting of patients with Crohn's Disease (CD);
- for each patient, of both the first subgroup and the second subgroup, isolating from the respective Raman spectrum the band in the wavenumber range of about 1550-1750 cm$^{-1}$;

- performing the steps c), d), e) and f) described above for each Raman spectrum;
- performing an analysis of a second ROC curve considering the ratios R of the first subgroup and of the second subgroup as database and calculating the second threshold value by Youden's index.

It was found that the second threshold value is greater than the first threshold value. The information obtained from a Raman scattering experiment is graphically depicted as a diagram (Raman spectrum) where the abscissas report the Raman shifts corresponding to the differences in energy between the vibrational levels involved in the transition with respect to the fundamental level, expressed in cm$^{-1}$ (wavenumber, keeping in mind the direct proportionality between the energy and the inverse of the wavelength of an electromagnetic radiation). Raman intensities proportional to the number of Stokes photons collected by the instrument detector are shown on the ordinates. The range of energies reported in a normal Raman spectrum can extend from a few tens of cm$^{-1}$ up to about 3500 cm$^{-1}$, a region in which almost all the fundamental molecular vibrations fall.

In particular, the Raman spectrum of the protein extract of a fecal sample can be acquired in a wavenumber range between 3500 cm$^{-1}$ and 300 cm$^{-1}$, preferably between 3300 cm$^{-1}$ and 400 cm$^{-1}$, even more preferably between 2500 cm$^{-1}$ and 500 cm$^{-1}$.

Raman spectroscopy can be performed by means of any Raman spectrometer having a laser source, such as, for example, a diode laser source, with a wavelength preferably, but not necessarily, equal to 780-790 nm.

The spectrometer is connected to a detector, preferably a charge-coupled device (CCD), possibly integrated with a monochromator.

The Raman spectrum can, for example, be acquired by performing a number of scans of the protein extract of a fecal sample between 25 and 35, with an exposure time between 30 and 90 seconds for each scan.

Between step a) and step b) it is preferable to perform a first fit of a region of the Raman spectrum in the range 1300-1800 cm$^{-1}$ by means of a predetermined profile for the sub-bands, preferably the Voigt profile, to isolate the band related to the amide-I in the range 1550-1750 cm$^{-1}$. To obtain said first fit, a first deconvolution is performed, leaving a minimum number of parameters free which characterize each sub-band, and an iterative best-fit procedure is started which therefore uses a minimum number of parameters.

Similarly, in order to perform the deconvolution of step d) (second curve-fitting), a minimum number of parameters characterizing each sub-band are left free and an iterative best-fit procedure is started which therefore uses a minimum number of parameters.

Before performing the Raman spectroscopy, the protein extract of the fecal sample is homogenized, preferably by centrifugation. In particular, a liquid extract is collected to homogenize the sample for Raman analysis.

An experimental study is described below, which allowed developing the method of the invention.

Materials and methods

A protein extract from fecal samples of 15 subjects with CD, 9 subjects with UC (both of these groups of subjects form the so-called IBD group) and 19 subjects for whom IBD was excluded (non-IBD group) was analyzed. In total, samples of 43 patients were analyzed, all of them in pediatric age. In particular, the IBD group had a median age of 15.6 ± 3.15 years, while the non-IBD group had a median age of 12.6 ± 5.17 years.

A fecal sample was collected for all subjects to assess the fecal levels of calprotectin. In the IBD group, the assessment was needed to assess disease activity (i.e., intensity of intestinal inflammation), in the non-IBD group the assessment was performed as part of the assessments to rule out the diagnosis of IBD. The patients in the IBD group, with CD or UC, had a different degree of disease activity based on calprotectin levels. Instead, the patients in the control group were those for whom intestinal inflammation was ruled out based on normal calprotectin levels (e.g., 100 mg/kg). From the same sample prepared for the ELISA test for fecal calprotectin, treated in a known manner with an extraction buffer solution in order to recover the protein content, a liquid extract of 100 μl was collected in order to homogenize the sample for Raman analysis.

The Raman measurements were performed using a DXR-Smart Raman spectrometer (Thermo Fisher Scientific) using a diode laser with an excitation wavelength of 785 nm. All the Raman spectra were acquired in the wavenumber range 400-3300 cm$^{-1}$, with a resolution of 1.9285 cm$^{-1}$ and irradiated with a laser power of 24 mW in output from a 50 μm pinhole aperture.

The detector connected to the spectrometer is a charge-coupled device (CCD) which, in addition to having very high sensitivity, allows simultaneously studying a broad spectral band, instead of a single wavelength at a time. In particular, the use of a CCD integrated with a monochromator allows obtaining, at once, the entire Raman spectrum with advantages in terms of experimental simplicity and speed of execution of the experiment.

The vials containing the fecal protein extract were placed in the sample holder thereof and the 180 degree sampling accessory of said DXR-Smart Raman spectrometer was used for the measurements. In order to obtain spectra with a high signal-to-noise ratio (S/R), each Raman spectrum was obtained after collecting 32 sample exposures and the duration of each exposure during the data collection was set equal to 60 s. The total acquisition time was therefore 32 minutes for each spectrum.

It proved preferable to fit a region of the Raman spectrum in the range 1300-1800 cm$^{-1}$ by means of a predetermined profile, preferably Voigt profiles, to isolate the band related to the amide-I in the range 1550-1750 cm$^{-1}$.

The experimental data, first in the range 1300-1800 cm$^{-1}$ and then in the range 1550-1750 cm$^{-1}$, were fitted using a multiple curve-fitting routine, for example provided in the PeakFit 4.0 software package (Systat Software, Inc., USA).

The fitting procedure adopted is a non-linear regression procedure, based on the least squares method, which adjusts the parameters in small steps in order to improve the goodness of the fit, minimizing the sum of squares of the vertical deviations $r^2 = \Sigma[y_i - f(x_i, a_1, a_2, ..., a_n)]^2$ of a set of n data points from a function f. In the previous relation, $y_i$ represents the ordinate, experimentally observed, corresponding to the abscissa $x_i$, while $f(x_i, a_1, a_2, ..., a_n)$ represents the ordinate, calculated using the function f depending on the n parameters $a_1, a_2, ..., a_n$, at the same abscissa $x_i$. The analysis of the second derivative of the selected band of the Raman spectrum was preliminarily applied to obtain, by evaluating the minima in the profiles obtained, a first indication of the minimum number of components of the band and the peak position thereof.

In the associated systems, the vibrational bands undergo both homogeneous and non-homogeneous broadening. Therefore, the Voigt profile, which is a convolution of a Lorentzian curve with a Gaussian curve, is particularly suitable for the analysis of the aforesaid bands. Considering that, the experimental data were deconvoluted into the minimum number of fit functions having a Voigt profile, defined as the following convolution of a Lorentzian curve with a Gaussian curve:

$$V(\omega) = \frac{\left\{ \int_{-\infty}^{+\infty} \frac{a_{Vgt} \exp\left(-x^2\right)dx}{\Gamma_{VL}^2 + \left[\left(\frac{\omega - \omega_{Vgt}}{\Gamma_{VG}}\right) - x\right]^2} \right\}}{\int_{-\infty}^{+\infty} \frac{\exp\left(-x^2\right)dx}{\Gamma_{VL}^2 + x^2}},$$

where

$\omega_{Vgt}$ is the center-frequency;

$a_{Vgt}$ is the amplitude;

$\Gamma_{VG}$ is the half-width related to the half-width at half-height of the Gaussian $\Gamma_G$, being

$$\Gamma_G = \Gamma_{VG} \sqrt{2\ln 2},$$

and $\Gamma_{VL}$ is the half-width related to the half-width at half-height of the Lorentzian $\Gamma_L$,

and is dependent on the ratio of the half-width at half-height of the Lorentzian $\Gamma_L$ with respect to said half-width $\Gamma_{VG}$, being $\Gamma_L = \Gamma_{VG} \cdot \Gamma_{VL}$.

All these four parameters, $a_{Vgt}$, $\omega_{Vgt}$, $\Gamma_{VG}$, $\Gamma_{VL}$, were left free to vary at the time of iteration. For each fit session, multiple iterations were performed until a convergent solution (best-fit) was reached, minimizing the value of $r^2$. The well-known problem of finding a local false minimum was overcome by running each nonlinear regression several times (20 times on average), each time providing a different set of initial parameter values. All the fits generated the same parameter values, with the same sum of squares, regardless of the initial values, and this confirmed not having encountered a false minimum.

As an alternative to the fit functions having a Voigt profile, other suitable fit functions could be used.

The ROC curve, as mentioned above, is a graph with the sensitivity on the ordinates and (1-specificity) on the abscissas, where the sensitivity represents the percentage of true positives, while (1-specificity) represents the percentage of true negatives. In this study, the sensitivity and specificity of the diagnostic test were determined by considering the positive and negative test in the two different groups (IBD subjects vs. non-IBD subjects). Sensitivity refers to the ability of Raman spectroscopy to correctly identify those patients in inflammatory phase. Instead, specificity refers to the ability of Raman spectroscopy to correctly identify patients without inflammation. The area under the ROC curve (AUC = Area under curve) represents the probability Pr (Y ≥ X), i.e., the probability of the marker value Y of a subject randomly selected from the ill population to be greater than the marker value X of a subject randomly selected from the healthy population.

The obtained AUC result is an effective mode to summarize the general diagnostic accuracy of the test. Values from 0 to 1 are used, where a value of 0 indicates a perfectly inaccurate test and a value of 1 reflects a perfectly accurate test. In general, an AUC value of 0.5 suggests no discrimination (i.e., the ability to diagnose patients with and without the disease or condition based on the test), an AUC value of 0.7 to 0.8 is considered acceptable, an AUC value of 0.8 to 0.9 is considered excellent, an AUC value greater than 0.9 is considered perfect.

On the other hand, since AUC serves as a measure of the overall diagnostic precision of a biomarker over all possible cut-points, the AUC cannot directly provide an optimal threshold or cut-point or cut-off point necessary to clinicians for diagnoses, e.g., to classify a subject as ill or healthy. For this reason, the cut-point was determined using the Youden index J, defined as J = sensitivity y + specificity y-1. This index represents the maximum vertical distance between the ROC curve and the reference represented by the diagonal, and is taken at the cut-point which optimizes the biomarker differentiation capacity when sensitivity and specificity are given equal weight. The Youden index J offers the optimal cut-off point and is simultaneously a direct measure of the diagnostic accuracy at the optimal cut-off point, i.e., the maximum correct general classification rate which a marker can achieve.

Results and discussion

The average Raman spectrum of the protein extract of the fecal samples of non-IBD patients was considered, obtained by averaging the spectra collected for all the subjects analyzed, previously normalized to the total area.

The spectrum highlighted some of the main vibrational modes typical of proteins, associated with the polypeptide backbone (amide bands) and the residual side of aromatic and non-aromatic amino acids.

The vibration modes of amide-I are clearly evident as a wide band centered at about 1650 $cm^{-1}$, and comprised in the wavenumber range of about 1550-1750 $cm^{-1}$. These vibration modes are mainly due to the stretching mode $v(C = O)$ and a small amount of out-of-phase stretching $v(C-N)$. The position of the amide-I band will depend on the conformations of the polypeptide backbone, e.g., on the type of secondary structure, and on the intramolecular and intermolecular hydrogen bond of the protein sample. Each type of secondary structure will have a typical stretching frequency $v(C=O)$, and this justifies the broadening of the band.

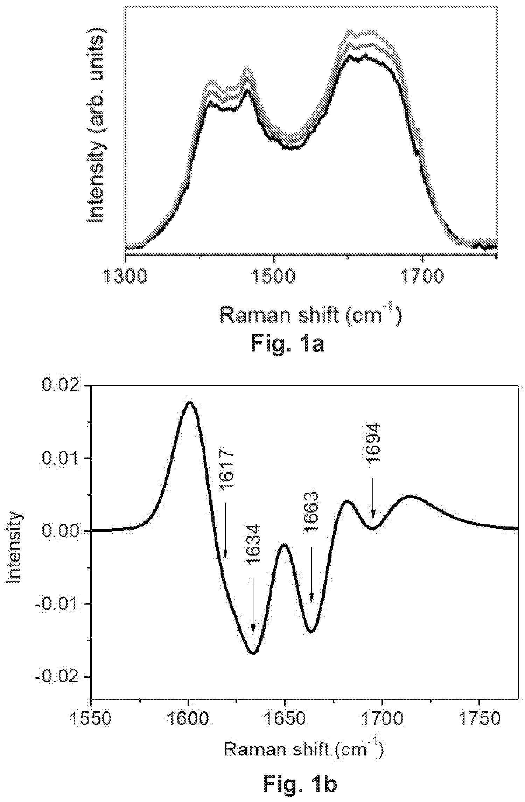

Figure 1a depicts the average Raman spectrum, in the range 1300-1800 $cm^{-1}$, of the fecal protein extract of non-IBD subjects (black line), subjects with CD (red line) and subjects with UC (green line). In fact, with the onset of the inflammatory disease, an evident increase in the spectral intensity in the broad band detected in the range 1300-1800 $cm^{-1}$ was observed.

The high quality of the spectra allowed us to quantitatively evaluate the spectral modifications possibly induced by the inflammatory state as a consequence of the changes in the protein secondary structures, focusing our attention in the amide-I region. In fact, this band is an excellent indicator for quantifying the secondary structure and conformational changes of the proteins, as a consequence of the role played by the amide part in the cross-linking.

To do this, the various protein secondary structures which contribute to the unresolved amide-I band must be distinguished, separating the corresponding components by means of second derivative, deconvolution and curve-fitting calculations.

In this sense, the main problem encountered in the spectra analysis is the overlapping of different components which contribute to the Raman vibrational profile observed in the range 1300-1800 $cm^{-1}$.

Preferably, a first curve-fitting can be performed only for the region or band of the Raman spectrum in the range 1300-1800 $cm^{-1}$ by means of, for example, Voigt profiles. The Voigt profile, described by a convolution of a Lorentzian curve with a Gaussian curve, is the most suitable in the case of associated systems, for which the vibrational bandwidth is affected by both homogeneous and non-homogeneous broadening.

After this step, all the sub-bands which fell outside the wavenumber range 1300-1800 $cm^{-1}$ were subtracted from the experimental profile, and a second more detailed curve-fitting was then performed only for the amide-I band, in the range 1550-1750 $cm^{-1}$. Also in this case it is preferable to perform the curve-fitting using Voigt profiles. The analysis of the second derivative of said amide-I band was advantageously applied to obtain, by evaluating the minima in the profiles obtained, a first indication of the positions of the peaks of the components of the amide-I band. Figure 1b shows an example of a profile obtained by calculating the second derivative of the experimental Raman spectrum of a non-IBD subject in the wavenumber range 1550-1750 $cm^{-1}$. Four main sub-bands were therefore recognized, centered respectively at about 1617 $cm^{-1}$, 1634 $cm^{-1}$, 1663 $cm^{-1}$ and 1694 $cm^{-1}$. Based on a comparison with the scientific literature these sub-bands were related to the

- vibration modes of the parallel β-sheet protein secondary structures, characterized by a strong hydrogen bond (indicative of protein aggregates) and which have the respective center-frequency at a wavenumber in the range 1610-1619 $cm^{-1}$, compatibly with the resolution of the Raman spectrometer;
- vibration modes of the lower frequency anti-parallel β-sheet protein secondary structures, which have the respective center-frequency at a wavenumber in the range 1620-1639 $cm^{-1}$, compatibly with the resolution of the Raman spectrometer;
- vibration modes of the disordered protein secondary structures of amide-I, which have the respective center-frequency at a wavenumber in the range 1660-1689 $cm^{-1}$, compatibly with the resolution of the Raman spectrometer;
- vibration modes of the higher frequency anti-parallel β-sheet protein secondary structures, which have the respective center-frequency at a wavenumber in the range 1690-1700 $cm^{-1}$, compatibly with the resolution of the Raman spectrometer.

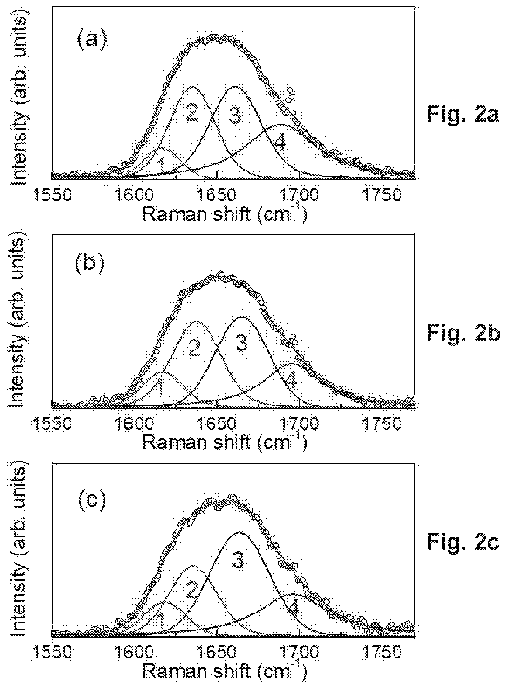

The results of a curve-fitting procedure obtained for a non-IBD subject, for a subject with CD and for a subject with UC are reported, as an example, in Figures 2a, 2b and 2c. The circles represent the experimental points; the line which follows the trend of the circles represents the best-fit; and curves 1, 2, 3 and 4 represent four sub-bands that are components of the amide-I band.

In particular, in the study case, the protocol used provided an optimal reproduction of the experimental data. In fact, for all the samples studied, the best-fit is characterized by $r^2 \sim 0.9999$.

Advantageously, the alterations of the protein network following the inflammatory state were monitored by evaluating the ratio R between the area of the sub-band $A_3$, associated with the vibration modes of the disordered protein secondary structures of amide-I, and the area of the sub-band $A_4$ associated with the vibration modes of the higher frequency anti-parallel β-sheet protein secondary structures. In particular, the ratio R is related to the non-reducible (trivalent)/reducible (bivalent) cross-linking ratio.

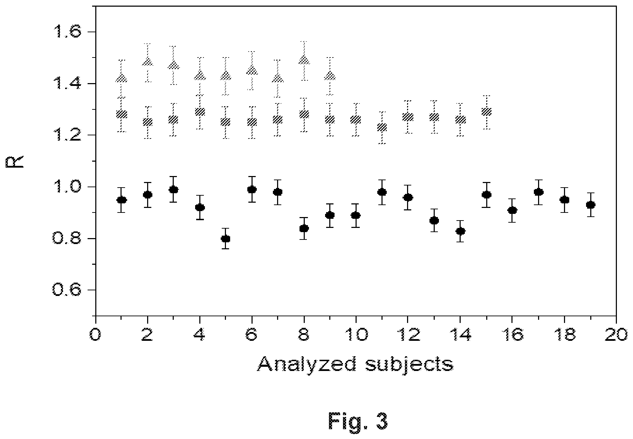

The R values obtained for all the subjects analyzed are reported in Figure 3.

It can be noted that active inflammation gives rise to a significant increase in the cross-linking ratio R, which passes from an average value of $0.93 \pm 0.05$ in the case of non-IBD subjects up to $1.26 \pm 0.06$ and $1.45 \pm 0.07$ in the case of subjects with CD and with UC disease, respectively. This increase is indicative of an increase, following inflammation, in the quantity of non-reducible trivalent cross-links at the expense of reducible bivalent cross-links, and/or following the reduced formation thereof. As a result, a more strongly interconnected protein network is conceivable, which clearly results in altered mechanical and functional properties. It could be argued that an increase in the ratio R in the amide-I band could also be attributed to conformational changes in proteins induced by factors other than inflammatory state, including aging, dehydration and radiological conditions. However, these factors do not influence the reported data, as the groups of subjects are very similar to each other, differing only with regard to intestinal inflammation.

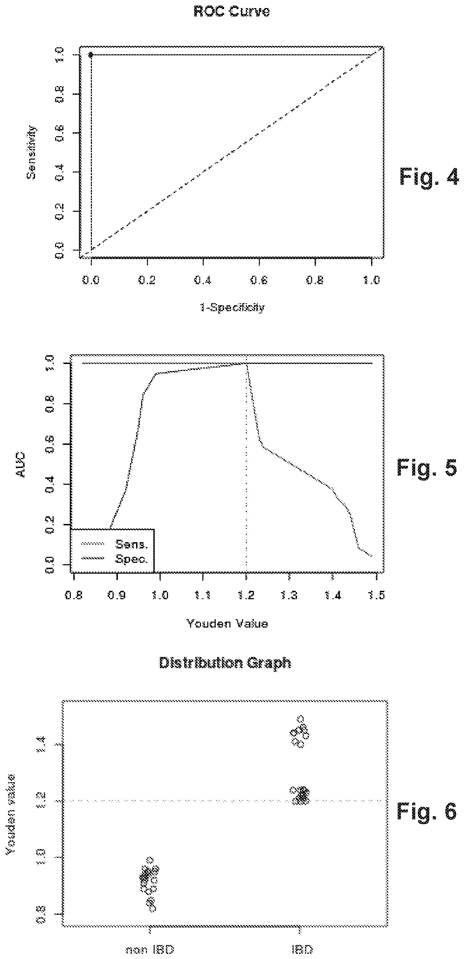

Figure 4 illustrates the ROC curve for IBD and non-IBD subjects (AUC = 1; 95% confidence range). Figure 5 shows the best cut-off point to maximize sensitivity and specificity (threshold value = 1.2). These curves show that the method of the invention is capable of clearly discriminating IBD subjects from non-IBD subjects. To highlight the above results, Figure 6 shows the distribution graph for IBD and non-IBD subjects.

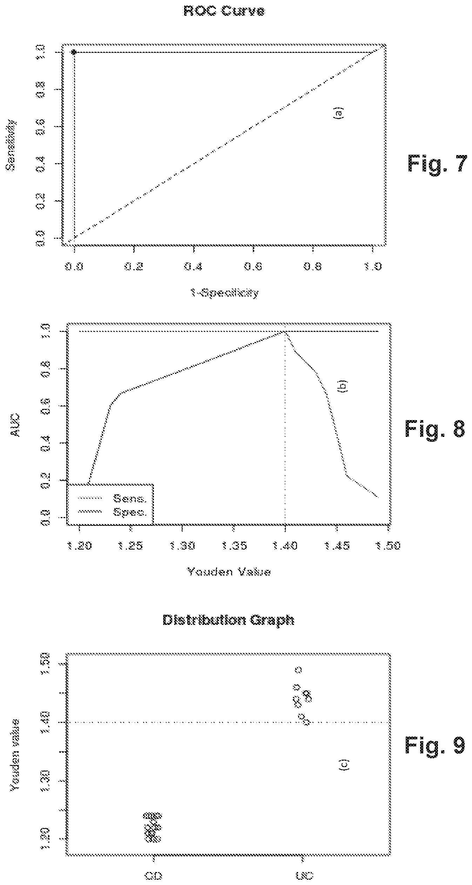

Considering the values obtained by the ratio R, the automatic differentiation between CD and UC was also advantageously performed in IBD patients. Figure 7 illustrates the ROC curve for IBD subjects (AUC = 1; 95% confidence range). Figure 8 shows the best cut-off point for maximizing sensitivity and specificity (threshold value = 1.4). These curves show that the method of the invention is also capable of clearly discriminating subjects with UC from subjects with CD considering the entire group of IBD subjects. To highlight the above results, Figure 9 shows the distribution graph for UC and CD subjects.

The results obtained with the method of the invention surprisingly demonstrated an optimal use of Raman spectroscopy as a diagnostic mode for the rapid detection of IBD and, among IBD subjects, for a clear and unambiguous distinction between CD and UC.

In addition to non-invasiveness, the proposed method offers significant advantages, including ease of use, experimental repeatability and speed of execution. Furthermore, the method is extremely low cost, with respect to the diagnostic techniques currently used which require special diagnostic kits, and is also non-destructive, since the sample can be stored and analyzed several times after some time, the only analysis limit being the degradation of the sample itself.

**Claims**

1. A diagnosis method for detecting inflammatory bowel diseases comprising Ulcerative Colitis (UC) and Crohn's Disease (CD), the method comprising the following steps:

   a) providing, as input data, a Raman spectrum of a protein extract of a fecal sample;
   b) isolating, from said Raman spectrum, the band in a wavenumber range of about 1550-1750 cm$^{-1}$, which defines the amide-I vibration modes;
   c) calculating the second derivative profile of said band of Raman spectrum, and identifying the minima of said profile;
   d) performing a deconvolution of said band, setting a number of sub-bands (1,2,3,4) equal to the number of minima of the profile of said second derivative, obtaining a number of sub-bands (1,2,3,4) equal to at least said number of minima, each sub-band having a respective center-frequency $\omega_i$ and a respective area $A_i$, with i = 1, 2,... n;
   e) associating the sub-bands (1,2,3,4) obtained in step d), based on the center-frequency $\omega$ thereof, with the corresponding vibration modes of the protein secondary structures comprising the vibration modes of the disordered protein secondary structures of amide-I and the vibration modes of the higher frequency anti-parallel β-sheet protein secondary structures;
   f) calculating the ratio R of the area of the sub-band, associated with the vibration modes of the disordered protein secondary structures of amide-I, with the area of the sub-band associated with the vibration modes of the higher frequency anti-parallel β-sheet protein secondary structures;
   g) verifying that the ratio R is greater than or equal to a first threshold value to confirm that the protein extract of the fecal sample belongs to a patient suffering from an inflammatory bowel disease between Ulcerative Colitis (UC) and Crohn's Disease (CD).

2. A method according to claim 1, wherein after step g) if said ratio R is greater than or equal to a second threshold value, the sample of fecal protein extract belongs to a patient suffering from Ulcerative Colitis (UC), while if said ratio R is lower than said second threshold value, the sample of fecal protein extract belongs to a patient suffering from Crohn's disease (CD).

3.  A method according to any one of the preceding claims, wherein in step d) in order to perform the deconvolution, a minimum number of parameters characterizing each sub-band is left free, and an iterative best-fit procedure is started, which thus uses said minimum number of parameters for each sub-band, preferably using a Voigt profile for each sub-band, each Voigt profile being mathematically expressed by a convolution of a Lorentzian curve with a Gaussian curve, and the characteristic parameters of which defining said minimum number of parameters comprise:

    the center-frequency $\omega_{Vgt}$;
    the amplitude $a_{Vgt}$;
    the half-width $\Gamma_{VG}$ related to the half-width at half-height of the Gaussian $\Gamma_G$, being

    $$\Gamma_G = \Gamma_{VG}\sqrt{2\ln 2}\ ,$$

    and the half-width $\Gamma_{VL}$ depending on the ratio of the half-width at half-height of the Lorentzian $\Gamma_L$ with respect to said half-width $\Gamma_{VG}$, being $\Gamma_L = \Gamma_{VG} \cdot \Gamma_{VL}$.

4.  A method according to any one of the preceding claims, wherein between step a) and step b) a fit of a region of the Raman spectrum between 1300-1800 cm$^{-1}$ is performed setting a predetermined profile, preferably the Voigt profile; preferably wherein, to obtain said fit, a deconvolution is performed, leaving a minimum number of parameters characterizing each sub-band free, and an iterative best-fit procedure is started, which thus uses said minimum number of parameters for each sub-band, preferably using a Voigt profile for each sub-band, each Voigt profile being mathematically expressed by a convolution of a Lorentzian curve with a Gaussian curve, and the characteristic parameters of which defining said minimum number of parameters comprise:

    the center-frequency $\omega_{Vgt}$;
    the amplitude $a_{Vgt}$;
    the half-width $\Gamma_{VG}$ related to the half-width at half height of the Gaussian $\Gamma_G$, being

    $$\Gamma_G = \Gamma_{VG}\sqrt{2\ln 2}\ ,$$

and the half-width $\Gamma_{VL}$ depending on the ratio of the half-width at half-height of the Lorentzian $\Gamma_L$ with respect to said half-width $\Gamma_{VG}$, being $\Gamma_L = \Gamma_{VG} \cdot \Gamma_{VL}$.

5.  A method according to any one of the preceding claims, wherein before performing the Raman spectroscopy, the fecal sample protein extract is homogenized, preferably by centrifugation.

6.  A method according to any one of the preceding claims, wherein said first threshold value can be defined by performing the following steps:

    - providing, as input data, a Raman spectrum of a fecal sample protein extract for each patient of a first known group of patients with Ulcerative Colitis (UC) or Crohn's Disease (CD) and for each patient of a second known group of healthy patients;
    - for each patient, of both the first group and second group, isolating from the respective Raman spectrum the band in the wavenumber range of about 1550-1750 cm$^{-1}$;
    - performing steps c), d), e) and f) for each Raman spectrum;
    - performing an analysis of a first ROC curve considering the ratios R of the first group and the second group as database and calculating the first threshold value by Youden's index.

7.  A method according to claim 6, wherein said second threshold value can be defined by performing the following steps:

    - providing, as input data, a Raman spectrum of a fecal sample protein extract for each patient of a first known subgroup of said first group consisting of patients with Ulcerative Colitis (UC) and for each patient of a second known subgroup of said first group consisting of patients with Crohn's Disease (CD);
    - for each patient, of both the first subgroup and the second subgroup, isolating from the respective Raman spectrum the band in the wavenumber range of about 1550-1750 cm$^{-1}$;
    - performing steps c), d), e) and f) for each Raman spectrum;
    - performing an analysis of a second ROC curve considering the ratios R of the first subgroup and the second subgroup as database and calculating the second threshold value by Youden's index.

8.  A method according to any one of claims 2 to 7, wherein said second threshold value is greater than said first threshold value.

9. A method according to any one of the preceding claims, wherein the Raman spectrum is acquired in a wavenumber range between 400 cm$^{-1}$ and 3300 cm$^{-1}$, preferably between 500 cm$^{-1}$ and 2000 cm$^{-1}$.

10. A method according to any one of the preceding claims, wherein a Raman spectrometer connected to a detector, preferably a CCD (charge-coupled device), is used to obtain the Raman spectrum of the fecal sample protein extract.

**Patentansprüche**

1. Diagnoseverfahren zum Nachweis von entzündlichen Darmerkrankungen, die Colitis ulcerosa (UC) und Morbus Crohn (CD) umfassen, wobei das Verfahren die folgenden Schritte umfasst:

   a) Bereitstellen eines Raman-Spektrums eines Proteinextrakts einer Fäkalprobe als Eingabedaten;
   b) Isolieren des Bandes in einem Wellenzahlbereich von etwa 1550-1750 cm$^{-1}$, das die Amid-I-Schwingungsmodi definiert, aus dem Raman-Spektrum;
   c) Berechnen des zweiten Ableitungsprofils des Bands des Raman-Spektrums und Identifizieren der Minima des Profils;
   d) Durchführen einer Entfaltung des Bandes, Einstellen einer Anzahl von Teilbändern (1,2,3,4) gleich der Anzahl von Minima des Profils der zweiten Ableitung, Erhalten einer Anzahl von Teilbändern (1,2,3,4) gleich mindestens der Anzahl von Minima, wobei jedes Teilband eine jeweilige Mittenfrequenz $\omega_i$ und einen jeweiligen Bereich $A_i$ aufweist, mit i = 1, 2,... n;
   e) Assoziieren der in Schritt d) erhaltenen Teilbänder (1,2,3,4) auf der Grundlage ihrer Mittenfrequenz $\omega$ mit den entsprechenden Schwingungsmoden der Protein-Sekundärstrukturen, die die Schwingungsmoden der ungeordneten Protein-Sekundärstrukturen von Amid-I und die Schwingungsmoden der höherfrequenten antiparallelen β-Faltblatt-Protein-Sekundärstrukturen umfassen;
   f) Berechnen des Verhältnisses R der Fläche des Teilbands, die mit den Schwingungsmodi der ungeordneten Protein-Sekundärstrukturen von Amid-I assoziiert ist, mit der Fläche des Teilbands, die mit den Schwingungsmodi der höherfrequenten antiparallelen β-Faltblatt-Protein-Sekundärstrukturen assoziiert ist;
   g) Überprüfen, ob das Verhältnis R größer oder gleich einem ersten Schwellenwert ist, um zu bestätigen, dass der Proteinextrakt der Fäkalprobe zu einem Patienten gehört, der an einer entzündlichen Darmerkrankung zwischen Colitis ulcerosa (UC) und Morbus Crohn (CD) leidet.

2. Verfahren nach Anspruch 1, wobei nach Schritt g), wenn das Verhältnis R größer oder gleich einem zweiten Schwellenwert ist, die Probe des Stuhlproteinextrakts zu einem Patienten gehört, der an Colitis ulcerosa (UC) leidet, während, wenn das Verhältnis R niedriger als der zweite Schwellenwert ist, die Probe des Stuhlproteinextrakts zu einem Patienten gehört, der an Morbus Crohn (CD) leidet.

3. Verfahren nach einem der vorhergehenden Ansprüche, wobei in Schritt d) zum Durchführen der Entfaltung eine Mindestanzahl von Parametern, die jedes Teilband charakterisieren, freigelassen wird und eine iterative Best-Fit-Prozedur gestartet wird, die somit die Mindestanzahl von Parametern für jedes Teilband verwendet, vorzugsweise unter Verwendung eines Voigt-Profils für jedes Teilband, wobei jedes Voigt-Profil mathematisch durch eine Faltung einer Lorentzschen Kurve mit einer Gaußschen Kurve ausgedrückt wird und die charakteristischen Parameter, von denen die Mindestanzahl von Parametern definiert wird, umfassen:

   die Mittenfrequenz $\omega_{Vgt}$;
   die Amplitude $a_{Vgt}$;
   die Halbwertsbreite $\Gamma_{VG}$ bezogen auf die Halbwertsbreite auf halber Höhe des Gaußschen $\Gamma_G$, wobei

   $$\Gamma_G = \Gamma_{VG}\sqrt{2\ln 2},$$

   und die Halbwertsbreite $\Gamma_{VL}$ in Abhängigkeit vom Verhältnis der Halbwertsbreite auf halber Höhe des Lorentzschen $\Gamma_L$ zur Halbwertsbreite $\Gamma_{VG}$, wobei $\Gamma_L = \Gamma_{VG} \cdot \Gamma_{VL}$.

4. Verfahren nach einem der vorhergehenden Ansprüche, wobei zwischen Schritt a) und Schritt b) eine Anpassung eines Bereichs des Raman-Spektrums zwischen 1300-1800 cm$^{-1}$ durchgeführt wird, indem ein vorbestimmtes Profil, vorzugsweise das Voigt-Profil, eingestellt wird; wobei vorzugsweise, um die Anpassung zu erhalten, eine Entfaltung durchgeführt wird, wobei eine Mindestanzahl von Parametern, die jedes Teilband charakterisieren, frei gelassen wird, und eine iterative Best-Fit-Prozedur gestartet wird, die somit die Mindestanzahl von Parametern für jedes Teilband verwendet, wobei vorzugsweise ein Voigt-Profil für jedes Teilband verwendet wird, wobei jedes Voigt-Profil mathematisch durch eine Faltung einer Lorentzschen Kurve mit einer Gaußschen Kurve ausgedrückt wird, und wobei die charakteristischen Parameter, die die Mindestanzahl von Parametern definieren, umfassen:

die Mittenfrequenz $\omega_{Vgt}$;
die Amplitude $a_{Vgt}$;
die Halbwertsbreite $\Gamma_{VG}$ bezogen auf die Halbwertsbreite auf halber Höhe des Gaußschen $\Gamma_G$, wobei

$$\Gamma_G = \Gamma_{VG}\sqrt{2\ln 2} \, ,$$

und die Halbwertsbreite $\Gamma_{VL}$ in Abhängigkeit vom Verhältnis der Halbwertsbreite auf halber Höhe des Lorentzschen $\Gamma_L$ zur Halbwertsbreite $\Gamma_{VG}$, wobei $\Gamma_L = \Gamma_{VG}\cdot\Gamma_{VL}$.

5. Verfahren nach einem der vorhergehenden Ansprüche, wobei vor der Durchführung der Raman-Spektroskopie der Fäkalprobenproteinextrakt homogenisiert wird, vorzugsweise durch Zentrifugation.

6. Verfahren nach einem der vorhergehenden Ansprüche, wobei der erste Schwellenwert durch Ausführen der folgenden Schritte definiert werden kann:

  - Bereitstellen eines Raman-Spektrums eines Fäkalprobenproteinextrakts als Eingabedaten für jeden Patienten einer ersten bekannten Gruppe von Patienten mit Colitis ulcerosa (UC) oder Morbus Crohn (CD) und für jeden Patienten einer zweiten bekannten Gruppe von gesunden Patienten;
  - für jeden Patienten, sowohl der ersten Gruppe als auch der zweiten Gruppe, Isolieren des Bandes im Wellenzahlbereich von etwa 1550-1750 cm$^{-1}$ aus dem jeweiligen Raman-Spektrum,
  - Durchführen der Schritte c), d), e) und f) für jedes Raman-Spektrum;
  - Durchführen einer Analyse einer ersten ROC-Kurve unter Berücksichtigung der Verhältnisse R der ersten Gruppe und der zweiten Gruppe als Datenbank und Berechnen des ersten Schwellenwerts durch den Youden-Index.

7. Verfahren nach Anspruch 6, wobei der zweite Schwellenwert durch Ausführen der folgenden Schritte definiert werden kann:

  - Bereitstellen eines Raman-Spektrums eines Fäkalprobenproteinextrakts als Eingabedaten für jeden Patienten einer ersten bekannten Teilgruppe der ersten Gruppe, bestehend aus Patienten mit Colitis ulcerosa (UC), und für jeden Patienten einer zweiten bekannten Teilgruppe der ersten Gruppe, bestehend aus Patienten mit Morbus Crohn (CD);
  - für jeden Patienten, sowohl der ersten Teilgruppe als auch der zweiten Teilgruppe, Isolieren des Bandes im Wellenzahlbereich von etwa 1550-1750 cm$^{-1}$ aus dem jeweiligen Raman-Spektrum,
  - Durchführen der Schritte c), d), e) und f) für jedes Raman-Spektrum;
  - Durchführen einer Analyse einer zweiten ROC-Kurve unter Berücksichtigung der Verhältnisse R der ersten Teilgruppe und der zweiten Teilgruppe als Datenbank und Berechnen des zweiten Schwellenwerts durch den Youden-Index.

8. Verfahren nach einem der Ansprüche 2 bis 7, wobei der zweite Schwellenwert größer als der erste Schwellenwert ist.

9. Verfahren nach einem der vorhergehenden Ansprüche, wobei das Raman-Spektrum in einem Wellenzahlbereich zwischen 400 cm$^{-1}$ und 3300 cm$^{-1}$, vorzugsweise zwischen 500 cm$^{-1}$ und 2000 cm$^{-1}$ erfasst wird.

10. Verfahren nach einem der vorhergehenden Ansprüche, wobei ein Raman-Spektrometer, das mit einem Detektor verbunden ist, vorzugsweise eine CCD (ladungsgekoppelte Vorrichtung), verwendet wird, um das Raman-Spektrum des Fäkalprobenproteinextrakts zu erhalten.

**Revendications**

1. Méthode de diagnostic pour détecter les maladies inflammatoires de l'intestin comprenant la colite ulcéreuse (UC) et la maladie de Crohn (CD), la méthode comprenant les étapes suivantes :

  a) fournir, comme données d'entrée, un spectre Raman d'un extrait protéique d'un échantillon fécal ;
  b) isoler, à partir dudit spectre Raman, la bande dans une gamme de nombre d'ondes d'environ 1550-1750 cm$^{-1}$, qui définit les modes de vibration de l'amide-I ;
  c) calculer le profil de la dérivée seconde de ladite bande de spectre Raman et identifier les minima dudit profil ;
  d) effectuer une déconvolution de ladite bande, en fixant un nombre de sous-bandes (1,2,3,4) égal au nombre de minima du profil de ladite dérivée seconde, en obtenant un nombre de sous-bandes (1,2,3,4) égal au moins audit nombre de minima, chaque sous-bande ayant une fréquence centrale respective $\omega_i$ et une surface respective $A_i$, avec i = 1, 2,... n ;
  e) associer les sous-bandes (1,2,3,4) obtenues à l'étape d), sur la base de leur fréquence centrale $\omega$, aux modes de vibration correspondants des structures secondaires protéiques comprenant les modes de vibration des structures secondaires protéiques désordonnées de l'amide-I et les modes de vibration des structures secondaires protéiques antiparallèles à feuillets β à plus haute fréquence ;

f) calculer le rapport R entre la surface de la sous-bande, associée aux modes de vibration des structures secondaires protéiques désordonnées de l'amide-I, et la surface de la sous-bande associée aux modes de vibration des structures secondaires protéiques antiparallèles à feuillets β à plus haute fréquence ;

g) vérifier que le rapport R est supérieur ou égal à une première valeur seuil pour confirmer que l'extrait protéique de l'échantillon fécal appartient à un patient souffrant d'une maladie inflammatoire de l'intestin entre la colite ulcéreuse (UC) et la maladie de Crohn (CD).

2. Méthode selon la revendication 1, dans laquelle, après l'étape g), si ledit rapport R est supérieur ou égal à une deuxième valeur seuil, l'échantillon d'extrait de protéine fécale appartient à un patient souffrant de colite ulcéreuse (UC), tandis que si ledit rapport R est inférieur à ladite deuxième valeur seuil, l'échantillon d'extrait de protéine fécale appartient à un patient souffrant de la maladie de Crohn (CD).

3. Méthode selon l'une quelconque des revendications précédentes, dans laquelle, à l'étape d), afin d'effectuer la déconvolution, un nombre minimum de paramètres caractérisant chaque sous-bande est laissé libre, et une procédure itérative de meilleur ajustement est lancée, qui utilise donc ledit nombre minimum de paramètres pour chaque sous-bande, de préférence en utilisant un profil de Voigt pour chaque sous-bande, chaque profil de Voigt étant mathématiquement exprimé par une convolution d'une courbe lorentzienne avec une courbe gaussienne, et dont les paramètres caractéristiques définissant ledit nombre minimum de paramètres comprennent :

la fréquence centrale $\omega_{Vgt}$ ;
l'amplitude $a_{Vgt}$ ;
la demi-largeur $\Gamma_{VG}$ liée à la demi-largeur à mi-hauteur de la gaussienne $\Gamma_G$, étant

$$\Gamma_G = \Gamma_{VG}\sqrt{2\ln 2}\,,$$

et la demi-largeur $\Gamma_{VL}$ dépendant du rapport de la demi-largeur à mi-hauteur de la lorentzienne $\Gamma_L$ par rapport à ladite demi-largeur $\Gamma_{VG}$, étant $\Gamma_L = \Gamma_{VG} \cdot \Gamma_{VL}$.

4. Méthode selon l'une quelconque des revendications précédentes, dans laquelle, entre l'étape a) et l'étape b), un ajustement d'une région du spectre Raman entre 1300-1800 cm$^{-1}$ est effectué en définissant un profil prédéterminé, de préférence le profil de Voigt ; de préférence dans laquelle, pour obtenir ledit ajustement, une déconvolution est effectuée, laissant libre un nombre minimum de paramètres caractérisant chaque sous-bande, et une procédure itérative de meilleur ajustement est lancée, qui utilise donc ledit nombre minimum de paramètres pour chaque sous-bande, de préférence utilisant un profil de Voigt pour chaque sous-bande, chaque profil de Voigt étant mathématiquement exprimé par une convolution d'une courbe lorentzienne avec une courbe gaussienne, et dont les paramètres caractéristiques définissant ledit nombre minimum de paramètres comprennent :

la fréquence centrale $\omega_{Vgt}$ ;
l'amplitude $a_{Vgt}$ ;
la demi-largeur $\Gamma_{VG}$ liée à la demi-largeur à mi-hauteur de la gaussienne $\Gamma_G$, étant

$$\Gamma_G = \Gamma_{VG}\sqrt{2\ln 2}\,,$$

et la demi-largeur $\Gamma_{VL}$ dépendant du rapport de la demi-largeur à mi-hauteur de la lorentzienne $\Gamma_L$ par rapport à ladite demi-largeur $\Gamma_{VG}$, étant $\Gamma_L = \Gamma_{VG} \cdot \Gamma_{VL}$.

5. Méthode selon l'une quelconque des revendications précédentes, dans laquelle, avant d'effectuer la spectroscopie Raman, l'extrait protéique d'échantillon fécal est homogénéisé, de préférence par centrifugation.

6. Méthode selon l'une quelconque des revendications précédentes, dans laquelle ladite première valeur seuil peut être définie en effectuant les étapes suivantes :

- fournir, comme données d'entrée, un spectre Raman d'un extrait protéique d'échantillon fécal pour chaque patient d'un premier groupe connu de patients atteints de colite ulcéreuse (UC) ou de la maladie de Crohn (CD) et pour chaque patient d'un second groupe connu de patients sains ;
- pour chaque patient, du premier groupe et du second groupe, isoler du spectre Raman respectif la bande dans la gamme de nombre d'ondes d'environ 1550-1750 cm$^{-1}$,
- effectuer les étapes c), d), e) et f) pour chaque spectre Raman ;
- effectuer une analyse d'une première courbe ROC en considérant les rapports R du premier groupe et du second groupe comme base de données et calculer la première valeur seuil par l'indice de Youden.

**7.** Méthode selon la revendication 6, dans laquelle ladite deuxième valeur seuil peut être définie en effectuant les étapes suivantes :

- fournir, comme données d'entrée, un spectre Raman d'un extrait protéique d'échantillon fécal pour chaque patient d'un premier sous-groupe connu dudit premier groupe composé de patients atteints de colite ulcéreuse (UC) et pour chaque patient d'un second sous-groupe connu dudit premier groupe composé de patients atteints de la maladie de Crohn (CD) ;
- pour chaque patient, du premier et du second sous-groupe, isoler du spectre Raman respectif la bande dans la gamme de nombre d'ondes d'environ 1550-1750 cm$^{-1}$,
- effectuer les étapes c), d), e) et f) pour chaque spectre Raman ;
- effectuer une analyse d'une seconde courbe ROC en considérant les rapports R du premier sous-groupe et du second sous-groupe comme base de données et calculer la deuxième valeur seuil par l'indice de Youden.

**8.** Méthode selon l'une quelconque des revendications 2 à 7, dans laquelle ladite deuxième valeur seuil est supérieure à ladite première valeur seuil.

**9.** Méthode selon l'une quelconque des revendications précédentes, dans laquelle le spectre Raman est acquis dans une gamme de nombre d'ondes comprise entre 400 cm$^{-1}$ et 3300 cm$^{-1}$, de préférence entre 500 cm$^{-1}$ et 2000 cm$^{-1}$.

**10.** Méthode selon l'une quelconque des revendications précédentes, dans laquelle un spectromètre Raman connecté à un détecteur, de préférence un CCD (dispositif à couplage de charge), est utilisé pour obtenir le spectre Raman de l'extrait protéique d'échantillon fécal.

Fig. 1a

Fig. 1b

Fig. 2a

Fig. 2b

Fig. 2c

Fig. 3

ROC Curve

Fig. 4

Fig. 5

Distribution Graph

Fig. 6

ROC Curve

Fig. 7

Fig. 8

Distribution Graph

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PENCE ISAAC J et al.** Clinical characterization of in vivo inflammatory bowel disease with Raman spectroscopy. *BIOMEDICAL OPTICS EXPRESS,* 04 January 2017, vol. 8 (2), 524-535